# EUROPÄISCHE PATENTSCHRIFT

(11) **EP 4 217 697 B1**
(45) Veröffentlichungstag und Bekanntmachung des Hinweises auf die Patenterteilung: **12.11.2025**
(21) Anmeldenummer: 21770016.0
(22) Anmeldetag: 02.09.2021
(51) Int. Cl.: G01K 17/00, G01N 1/28, G01N 33/38, B01F 31/20, B01F 31/86, G01N 25/48

(54) **MISCHVORRICHTUNG ZUR VORBEREITUNG EINER PROBE**
MIXING DEVICE FOR PREPARING A SAMPLE
DISPOSITIF DE MÉLANGE POUR LA PRÉPARATION D'UN ÉCHANTILLON

(30) Priorität: 23.09.2020 DE 102020211917; 23.09.2020 BE 202005651
(43) Veröffentlichungstag der Anmeldung: 02.08.2023
(73) Patentinhaber: thyssenkrupp Polysius GmbH, 59269 Beckum (DE); thyssenkrupp AG, 45143 Essen (DE)
(72) Erfinder: TEUTENBERG, Reinhard, 59423 Unna (DE); ENDERS, Michael, 48143 Münster (DE)
(74) Vertreter: thyssenkrupp Intellectual Property GmbH
(86) Internationale Anmeldenummer: PCT/EP2021/074211
(87) Internationale Veröffentlichungsnummer: WO 2022/063539

(56) Entgegenhaltungen:
- DE-A1- 102014 018 489
- US-A- 5 365 788
- US-A1- 2006 231 653
- US-A1- 2020 150 005

## Beschreibung

Die Erfindung betrifft eine Mischvorrichtung zur Vorbereitung eines Probematerials auf eine kalorimetrische Messung, sowie eine Anlage zur Bestimmung der Reaktivität eines Probematerials und ein Verfahren zum Ermitteln der Reaktivität eines Probematerials.

Anorganische Bindemittel werden in der Größenordnung von 4Gt jährlich in der Bauindustrie eingesetzt. Die Zusammensetzung der Bindemittel hat sich in den letzten Jahrzehnten verändert. An die Stelle traditioneller Zemente auf der Basis von Portlandzementklinker und Sulfatträger sind vielfach nachhaltige, kostengünstig herstellbare und hinsichtlich der Anwendungseigenschaften optimierte Kompositzemente aus Klinker, Zumahlstoffen und Sulfatträger getreten.

Vor dem Hintergrund steigender Komplexität der Bindemittelzusammensetzung, der notwendigen Anpassung der Bindemittelfeinheit und der Anwendungseigenschaften sind der finanzielle Aufwand und der Zeitaufwand bei der Produktoptimierung und der Produktentwicklung angestiegen. Die Zielgrößen bei einer Produktoptimierung und der Produktentwicklung umfassen dabei zum Beispiel die Verarbeitbarkeit, das Erstarrungsverhalten und die Festigkeitsentwicklung. Abschließend muss die Leistungsfähigkeit des Bindemittels in der Hauptanwendung Beton untersucht werden. Der hohe Materialbedarf für Betonuntersuchungen erfordert eine frühe Vorauswahl geeigneter Bindemittelzusammensetzungen und geeigneter Bindemittelfeinheitsbereiche. Die üblichen in der Baustoffindustrie angewandten physikalischen und in der EN 196 und EN 206 beschriebenen Analyseverfahren ermöglichen aufgrund des erheblichen Materialbedarfs lediglich eine Analyse einer geringen Probezahl und sind zudem zum Beispiel aufgrund der Prüfalter bis zu 28 Tagen sehr zeitaufwendig in ihrer Durchführung. Die Ermittlung der Auswirkung verschiedenster Parametervariationen auf die Reaktivität des Bindemittels ist daher sehr zeitaufwendig.

Kalorimeter werden üblicherweise manuell bedient. Für Routinemessungen muss zudem eine Stabilisierung einer Basislinie abgewartet werden, was den Messbeginn weiter verzögert. Die Vorbereitung einer Probe erfolgt üblicherweise ebenfalls manuell, wobei der Probendurchsatz sehr gering ist. Des Weiteren führen manuelle Vorbereitungen der Probe zu Ungenauigkeiten und schlecht vergleichbaren Daten mit einer geringen Reproduzierbarkeit. US 2020/0150005 A1 befasst sich mit der Aufbereitung von Proben. Es handelt sich dabei, z.B., um die Aufbereitung von Zementproben, die einem Testlabor zugeschickt werden können. Das Gerät der US'005 verwendet kleine Kugeln aus Metall oder Keramik um die Probe zu mahlen. Das Gerät umfasst einen Rahmen und eine Aufnahmeeinrichtung für die Probe, die durch einen Vibrator in eine oszillierende Bewegung versetzt wird.

Davon ausgehend ist es Aufgabe der vorliegenden Erfindung, eine Vorrichtung bereitzustellen, mit welcher eine automatisierte Vorbereitung und Handhabung des Probematerials möglich ist, sodass der Probendurchsatz und die Messgenauigkeit erhöht werden.

Diese Aufgabe wird erfindungsgemäß durch eine Mischvorrichtung mit den Merkmalen des unabhängigen Vorrichtungsanspruchs 1 und durch ein Verfahren mit den Merkmalen des unabhängigen Verfahrensanspruchs 12 gelöst. Vorteilhafte Weiterbildungen ergeben sich aus den abhängigen Ansprüchen.

Eine Mischvorrichtung zur Vorbereitung eines Probematerials auf eine kalorimetrische Messung umfasst nach einem ersten Aspekt einen Rahmen und eine mit dem Rahmen verbundene Aufnahmeeinrichtung zur Aufnahme eines mit dem Probematerial gefüllten Behälters. Die Aufnahmeeinrichtung ist mit einem oder beispielsweise einer Mehrzahl von Vibratoren zur Vibration der Aufnahmeeinrichtung verbunden und zwischen der Aufnahmeeinrichtung und dem Rahmen ist ein Schwingungsdämpfer angebracht. Der Vibrator ist pneumatisch betrieben, wobei zumindest zwei Vibratoren vorgesehen sind und wobei einer der Vibratoren zur Erzeugung einer horizontalen und der andere der Vibratoren zur Erzeugung einer vertikalen Schwingung ausgebildet ist.

Das Probematerial ist beispielsweise ein Klinker oder ein hydraulisches Bindemittel mit unterschiedlichen Zusammensetzungen verschiedener Werkstoffkomponenten, wie beispielsweise Klinker, Sulfatträger oder Zumahlstoffe aufweisen. Zumahlstoffe sind beispielsweise Hüttensand, Flugasche, Puzzolan, Kalkstein oder kalzinierter Ton. Das Probematerial ist vorzugsweise gemahlen, insbesondere pulverförmig. Es ist ebenfalls denkbar, dass das Probematerial eine Anregerflüssigkeit, wie beispielsweise destilliertes Wasser oder Wasser und ein Zement oder Betonzusatzmittel oder auch einen alkalischen Aktivator umfasst.

Bei einer kalorimetrischen Messung des Probematerials wird vorzugsweise die Reaktivität des Probematerials ermittelt. Vorzugsweise wird dazu die von dem Probematerial emittierte Wärmemenge pro Zeit nach Zugabe einer Anregerflüssigkeit ermittelt. Die kalorimetrische Messung erfolgt vorzugsweise isotherm.

Die Rahmen dient der Abstützung der Aufnahmeeinrichtung und vorzugsweise der Montage der Aufnahmeeinrichtung beispielsweise an einem Tisch oder einer weiteren Anlage zur Bearbeitung des Probematerials. Der Rahmen umschließt die Aufnahmeeinrichtung der Mischvorrichtung vorzugsweise zumindest teilweise und ist insbesondere kastenförmig ausgebildet. An dem Rahmen ist zusätzlich zu der Aufnahmeeinrichtung der zumindest eine Schwingungsdämpfer angebracht. Insbesondere weist die Mischvorrichtung zumindest zwei Schwingungsdämpfer auf, die jeweils zwischen dem Rahmen und der Aufnahmeeinrichtung angebracht sind und sich insbesondere einander gegenüberliegen. Vorzugsweise ist die Aufnahmeeinrichtung nicht direkt mit dem Rahmen, sondern ausschließlich über den Schwingungsdämpfer mit dem Rahmen verbunden.

Der von der Mischvorrichtung umfasste Behälter ist vorzugsweise verschließbar, sodass das Probematerial vollständig innerhalb des Behälters aufgenommen ist. Die Aufnahmeeinrichtung weist beispielsweise eine insbesondere zylinderförmige Aussparung auf, in die der Behälter eingeführt wird. Der Behälter ist beispielsweise von oben oder von unten in die Aussparung einführbar.

An der Aufnahmeeinrichtung ist vorzugsweise zumindest ein Vibrator derart befestigt, dass er die Aufnahmeeinrichtung mit einer Vibration, insbesondere einer gerichteten Schwingung beaufschlagt. Vorzugsweise ist der Vibrator nicht direkt an dem Rahmen befestigt. Der Vibrator ermöglicht ein automatisches Mischen des Probematerials in dem Behälter, wobei der Schwingungsdämpfer dafür sorgt, dass die Schwingungen des Vibrators lediglich auf die Aufnahmeeinrichtung, vorzugsweise auf den Behälter, und nicht oder nur geringfügig auf den Rahmen übertragen werden.

Gemäß einer ersten Ausführungsform weist die Aufnahmeeinrichtung eine Temperiereinrichtung zum Kühlen oder Erwärmen des Behälters auf. Die Temperiereinrichtung dient vorzugsweise zum Kühlen und/ oder Heizen der Aufnahmeeinrichtung und/ oder des Behälters. Dies ermöglicht eine automatische Vorwärmung oder Kühlung des Materials vor der Ermittlung der Reaktivität in dem Kalorimeter. Zur optimalen Messung der Reaktivität ist eine bestimmte Temperatur des Probematerials vorteilhaft.

Der Vibrator ist pneumatisch betrieben. Erfindungsgemäß sind zumindest zwei Vibratoren vorgesehen, wobei einer der Vibratoren zur Erzeugung einer horizontalen und der andere der Vibratoren zur Erzeugung einer vertikalen Schwingung ausgebildet und eingerichtet ist. Vorzugsweise ist einer der Vibratoren derart ausgebildet, dass er ausschließlich eine vertikal gerichtete Schwingung erzeugt, wobei der andere der Vibratoren insbesondere derart ausgebildet ist, dass er ausschließlich eine horizontal gerichtete Schwingung erzeugt. Vorzugsweise sind die Vibratoren derart ausgebildet, dass sie jeweils eine Schwingung erzeugten, die orthogonal zu der Schwingung des jeweils anderen Vibrators ist. Die Vibratoren sind derart mit der Aufnahmeeinrichtung verbunden, dass sie diese mit der Schwingung beaufschlagen. Dies ermöglicht eine gezielte Mischung des Probematerials, wobei der Anteil an horizontal und vertikal ausgerichteter Schwingung individuell eingestellt werden kann. Die Vibratoren können zudem mit innenliegenden Prallplatten ausgestattet werden, so dass neben den Schwingungen zur verbesserten Durchmischung auch ein Schlagen/Klopfen erzeugt wird. Zudem bleibt das Material nicht an den Wandungen des Behälters (oberhalb des Füllungsgrades) haften.

Gemäß einer weiteren Ausführungsform weist die Temperiereinrichtung Leitungen zum Leiten eines Fluids, insbesondere einer Flüssigkeit, auf. Vorzugsweise erstrecken sich die Leitungen durch die Aufnahmeeinrichtung insbesondere nahe der Oberfläche, an der der Behälter anliegt. Vorzugsweise sind die Leitungen zumindest teilweise in dem Behälter angeordnet. Bei dem Fluid handelt es sich beispielsweise um Wasser zur Kühlung und/ oder Erwärmung der Aufnahmeeinrichtung und des Behälters. Dies ermöglicht eine zuverlässige Temperierung des Probematerials vor der Messung der Reaktivität in dem Kalorimeter.

Gemäß einer weiteren Ausführungsform weist die Mischvorrichtung eine Temperaturmesseinrichtung zur Ermittlung der Temperatur innerhalb des Behälters und/ oder der Aufnahmeeinrichtung auf. Die Temperaturmesseinrichtung ist vorzugsweise in dem Behälter oder der Aufnahmeeinrichtung angebracht.

Gemäß einer weiteren Ausführungsform weist die Mischvorrichtung eine Steuerungs-/ Regelungseinrichtung auf, die mit dem Vibrator und/ oder der Temperiereinrichtung verbunden ist und derart ausgebildet ist, dass sie die Temperatur der Temperiereinrichtung und/ oder die Schwingungsamplitude und/ oder die Schwingungsfrequenz des Vibrators steuert/ regelt. Zudem lassen sich die Vibratoren zur Einbringung der horizontalen und der vertikalen Schwingung vorzugsweise getrennt steuern. Die Zyklen und die Reihenfolge der Ansteuerung kann variiert werden (z.B. erst Ansteuerung des Vibrators zur Einbringung der horizontalen Schwingung und anschließend Ansteuerung des Vibrators zur Einbringung der vertikalen Schwingung, der beiden Schwingungen gleichzeitig). Vorzugsweise ist an der Steuerungs-/ Regelungseinrichtung manuell oder automatisch ein vorabbestimmter Sollwert der Temperatur, der Amplitude und/ oder der Frequenz einstellbar. Vorzugsweise wird der Sollwert der Temperatur, der Amplitude und/ oder der Frequenz in Abhängigkeit der Zusammensetzung des Probewerkstoffes eingestellt. Bei dem Sollwert kann es sich auch um einen zeitlichen Sollwertverlauf handeln.

Gemäß einer weiteren Ausführungsform ist die Steuerungs-/Regelungseinrichtung mit der Temperaturmesseinrichtung verbunden und derart ausgebildet, dass sie die Temperatur der Temperiereinrichtung und/ oder die Schwingungsamplitude und/ oder die Schwingungsfrequenz des Vibrators in Abhängigkeit der ermittelten Temperatur steuert/ regelt. Die Temperaturmesseinrichtung ist vorzugsweise zur Übermittlung der ermittelten Temperatur der Aufnahmeeinrichtung und/ oder des Behälters mit der Steuerungs- / Regelungseinrichtung verbunden. Die Steuerungs-/ Regelungseinrichtung ist vorzugsweise derart ausgebildet, dass sie die mittels der Temperaturmesseinrichtung ermittelte Temperatur der Aufnahmeeinrichtung und/ oder des Behälters mit dem Sollwert vergleicht und bei einer Abweichung der Temperatur von dem Sollwert, die Temperatur der Temperiereinrichtung entsprechend erhöht oder verringert.

Gemäß einer weiteren Ausführungsform weist die Aufnahmeeinrichtung eine Klemmvorrichtung zum Fixieren des Behälters auf. Die Klemmvorrichtung weist gemäß einer weiteren Ausführungsform Klemmbacken und einen Pneumatikzylinder auf, der zur Bewegung der Klemmbacken mit diesen verbunden ist. Vorzugsweise wird der Behälter zwischen den Klemmbacken in der Aufnahmeeinrichtung fixiert.

Gemäß einer weiteren Ausführungsform ist die Steuerungs-/ Regelungseinrichtung mit dem Pneumatikzylinder zu dessen Steuerung/ Regelung verbunden. Dies ermöglicht ein automatisches Fixieren des Behälters in der Aufnahmeeinrichtung.

Gemäß einer weiteren Ausführungsform weist die Mischvorrichtung einen pneumatischen Klopfer auf, der an dem Behälter oder in der Aufnahmeeinrichtung angeordnet ist. Bei dem Klopfer handelt es sich beispielsweise um einen beispielsweise mittels einer Feder vorgespannten, insbesondere horizontal angeordneten pneumatischen Zylinder, mit dem impulsartige Stöße ausgeführt werden können, die auf den Behälter wirken. Vorzugsweise ist der Klopfer mit der Steuerungs-/ Regelungseinrichtung verbunden, wobei diese den Klopfer in Abhängigkeit der Zusammensetzung, insbesondere der. Art des Probematerials oder der Wassermenge steuert/ regelt.

Die Erfindung umfasst auch eine Anlage zur Bestimmung der Reaktivität eines Probematerials aufweisend eine Dosiereinrichtung zum Dosieren des Probematerials und/ oder vorzugsweise einer Anregerflüssigkeit in einen Behälter, eine Mischvorrichtung wie vorangehend beschrieben, zur Aufnahme des Behälters und zum Mischen des Probematerials in dem Behälter und ein Kalorimeter zur Ermittlung der Reaktivität des gemischten Probematerials. Die Anlage umfasst vorzugsweise eine weitere eine Dosiereinrichtung zum Dosieren eines Fluids, wie beispielsweise Wasser, in den Behälter.

Ein Kalorimeter, insbesondere ein isothermes Wärmeflusskalorimeter. Ist derart ausgebildet, dass sie die von dem Probewerkstoff abgegebene Reaktionswärme ermittelt. Die freigegebene Reaktionswärme und der Verlauf der Wärmeabgabe über die Zeit sind charakteristisch für die Reaktivität eines Probewerkstoffs, insbesondere eines Bindemittels. Die Zugabe einer Anregerflüssigkeit, wie beispielsweise Wasser, zu den Werkstoffkomponenten startet den Hydratationsprozess, wobei die in den Werkstoffkomponenten gespeicherte Energie in Form von Reaktionswärme freigesetzt wird. Die kalorimetrische Messeinrichtung ermöglicht eine einfache und schnelle Ermittlung der Reaktivität des Probewerkstoffs.

Die Erfindung umfasst auch ein Verfahren zum Ermitteln der Reaktivität eines Probematerials aufweisend die Schritte:
- Dosieren des Probematerials in einen Behälter,
- vorzugsweise Dosieren des Fluids oder eines weiteren Pulvers, wie beispielsweise einen Sulfatträger in den gleichen Behälter und Verschließen des Behälters,
- Fixieren des Behälters in der Aufnahmeeinrichtung einer Mischvorrichtung wie vorangehend beschrieben,
- Vibrieren der Aufnahmeeinrichtung mittels der Vibratoren der Mischvorrichtung,
- Zuführen des Behälters zu einem Kalorimeter und
- Ermitteln eines Kalorimetrischen Wertes des Probematerials in dem Behälter.

Zusätzlich zu dem Probematerial wird gemäß einer Ausführungsform eine Anregerflüssigkeit in den Behälter dosiert. Anschließend wird der Behälter vorzugsweise verschlossen. Das Vibrieren der Aufnahmeeinrichtung erfolgt vorzugsweise nach einem vorgegebenen Sollwertverlauf der Amplitude und/ oder der Frequenz der mittels des Vibrators erzeugten Schwingung.

### Beschreibung der Zeichnungen

Die Erfindung ist nachfolgend anhand mehrerer Ausführungsbeispiele mit Bezug auf die beiliegenden Figuren näher erläutert.
- Fig. 1: zeigt eine schematische Darstellung einer Mischvorrichtung in einer perspektivischen Ansicht gemäß einem Ausführungsbeispiel.
- Fig. 2: zeigt eine schematische Darstellung einer Anlage zur Bestimmung der Reaktivität eines hydraulischen Bindemittels mit einer Mischvorrichtung aus Fig. 1 gemäß einem Ausführungsbeispiel.

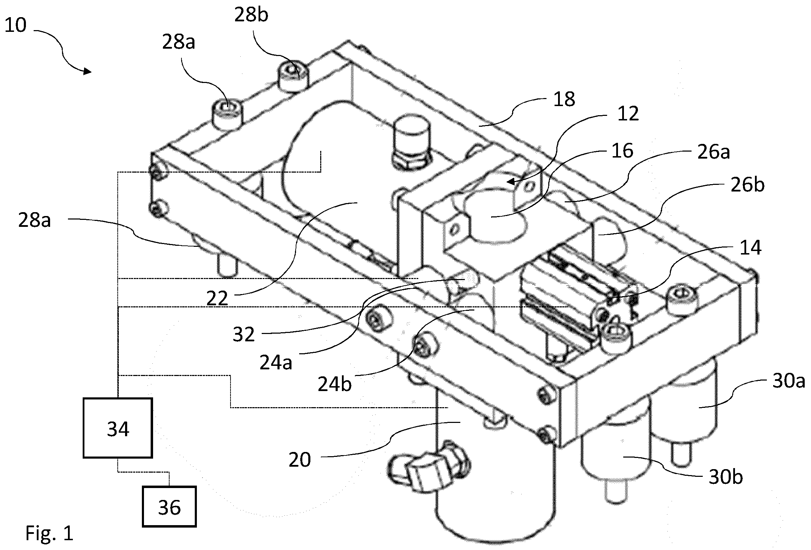

In Fig. 1 zeigt eine Mischvorrichtung 10 zum Mischen eines Probematerials. Bei dem Probematerial handelt es sich beispielsweise um gemahlenes Rohmehl, gemahlener Zement oder Zementklinker, vorzugsweise als feinkörniges Pulver, wobei diese beispielsweise mit einem Sulfatträger vermengt sind. Die Mischvorrichtung 10 dient zur Vorbereitung des Probematerials auf eine kalorimetrische Messung Bei einer kalorimetrischen Messung handelt es sich insbesondere um eine Ermittlung der von dem Probematerial abgegebenen Wärme nach Zugabe einer Anregerflüssigkeit, wie beispielsweise Wasser oder destilliertes Wasser. zu dem Probematerial. Die abgegebene Wärme ist ein Maß für die in dem Probematerial gespeicherte Energie.

Die Mischvorrichtung 10 weist eine Aufnahmeeinrichtung 12 zur Aufnahme eines mit Probematerial gefüllten Behälters auf. Bei der Aufnahmeeinrichtung 12 handelt es sich beispielsweise um eine Kapsel oder eine Mischkammer. Die Aufnahmeeinrichtung 12 weist in dem Ausführungsbeispiel der Fig. 1 beispielhaft eine zylinderförmige, nach oben und/ oder unten offene Aussparung zur Aufnahme des Behälters auf. Es ist ebenfalls denkbar, dass die Aufnahmeeinrichtung 12 den Behälter vollständig umschließt und beispielsweise eine Klappe zum Öffnen der Aufnahmeeinrichtung 12 aufweist. Vorzugsweise umfasst die Aufnahmeeinrichtung 12 eine Klemmvorrichtung mittels welcher der mit dem Probematerial gefüllte Behälter in der Aufnahmeeinrichtung 12 fixierbar ist. Beispielhaft weist die Klemmvorrichtung einen Pneumatikzylinder 14 und vorzugsweise in Fig. 1 nicht sichtbare Klemmbacken auf, wobei diese mittels des Pneumatikzylinders 14 bewegbar sind.

Die Mischvorrichtung 10 weist des Weiteren einen Rahmen 18 auf. Der Rahmen 18 umschließt die Aufnahmeeinrichtung 12 der Mischvorrichtung 12 zumindest teilweise. Beispielhaft ist der Rahmen 18 kastenförmig ausgebildet. An der Aufnahmeeinrichtung 12 sind Vibratoren 20, 22 angebracht. Erfindungsgemäß sind zwei Vibratoren an der Aufnahmeeinrichtung 12 angebracht, wobei ein erster Vibrator 20 derart ausgebildet und angeordnet ist, dass er die Aufnahmeeinrichtung 12 mit einer vertikalen Schwingung beaufschlagt und ein zweiter Vibrator 22 derart ausgebildet und angeordnet ist, dass er die Aufnahmeeinrichtung 12 mit einer horizontalen Schwingung beaufschlagt. Die Vibratoren 20, 22 sind pneumatisch angetrieben. Insbesondere sind die Vibratoren 20, 22 mit einer Steuerungs-/Regelungseinrichtung 34 zur Steuerung/ Regelung der Frequenz und/ oder der Amplitude der Schwingungen verbunden. Es ist ebenfalls denkbar, dass an der Aufnahmeeinrichtung 12 mehr als zwei Vibratoren, beispielsweise vier, sechs oder acht Vibratoren, angebracht sind. Außerdem kann auch lediglich ein Vibrator an der Aufnahmeeinrichtung 12 angebracht sein.

Die Aufnahmeeirichtung 12 ist insbesondere über Schwingungsdämpfer 24a,b und 26a,b mit dem Rahmen 18 verbunden. Jeder Schwingungsdämpfer 24, 26 weist beispielhaft zwei Dämpfereinheiten auf, die parallel zueinander und gleichwirkend angeordnet sind. Vorzugsweise sind an der Aufnahmeeinrichtung 12 zwei Schwingungsdämpfer 24, 26 angebracht, die insbesondere jeweils mit ihrem einen Ende an dem Rahmen 18 und mit dem jeweiligen anderen Ende an der Aufnahmeeinrichtung 12 angebracht sind. Die Schwingungsdämpfer 24, 26 dienen der Befestigung der Aufnahmeeinrichtung 12 an dem Rahmen, wobei die Schwingung der Aufnahmeeinrichtung 12 kaum oder gar nicht auf den Rahmen 18 übertragen wird. Die Schwingungsdämpfer 24, 26 sind vorzugsweise an einander gegenüberliegenden Seitenfläche der Aufnahmeeinrichtung 12 angebracht. Optional sind an dem Rahmen 18 zwei weitere Schwingungsdämpfer 28a,b, 30a,b angebracht, über welche der Rahmen 18 an einem weiteren Gegenstand, wie beispielsweise einem Tisch oder Gestell, befestigbar ist. Die Schwingungsdämpfer sind jeweils an einander gegenüberliegenden Enden des Rahmens 18 angebracht und mit ihrem einen Ende an dem Rahmen 18 und mit ihrem anderen Ende an einem in Fig. 1 nicht dargestellten Gegenstand, wie Tisch oder Gestell angebracht. Die Schwingungsdämpfer 28, 30 verhindern eine Übertragung der Schwingung der Aufnahmeeinrichtung 12 auf einen Gegenstand, wie einen Tisch oder ein Gestell, außerhalb des Rahmens 18.

Die Aufnahmeeinrichtung 12 weist beispielsweise eine Temperiereinrichtung 32 zur Einstellung der Temperatur der Aufnahmeeinrichtung 12. Vorzugsweise ist die Temperiereinrichtung 32 mit der Steuerungs-/ Regelungseinrichtung 34 verbunden, sodass diese die Temperatur der Temperiereinrichtung 32, insbesondere der Aufnahmeeinrichtung 12, steuert/ regelt. Die Temperiereinrichtung 32 umfasst beispielsweise Leitungen zum Leiten eines Kühl-/ Wärmemediums. Vorzugsweise weist die Aufnahmeeinrichtung 12 eine Temperaturmesseinrichtung 36 zur Ermittlung der Temperatur der Aufnahmeeinrichtung 12 auf. Die Temperaturmesseinrichtung 36 ist vorzugsweise zur Übermittlung der ermittelten Temperatur mit der Steuerungs-/Regelungseinrichtung 34 verbunden. Es ist ebenfalls denkbar, dass die Temperaturmesseinrichtung 36 in einem Behälter zur Aufnahme des Probematerials angeordnet ist. Die Steuerungs-/Regelungseinrichtung 34 ist vorzugsweise derart ausgebildet, dass sie die mittel der Temperaturmesseinrichtung 36 ermittelte Temperatur mit einem vorabbestimmten Sollwert oder Sollbereich vergleicht und bei einer Abweichung der Temperatur von Sollwert oder dem Sollbereich, die mittels der Temperiereinrichtung 32 die Temperatur der Aufnahmeeinrichtung 12 erhöht oder verringert. Vorzugsweise wird bei einem Unterschreiten des Sollwertes oder des Sollbereichs die Temperatur erhöht und bei einem Überschreiten des Sollwertes oder des Sollbereichs die Temperatur verringert. Der Sollwert beträgt beispielsweise etwa 20°C bis 45°, vorzugsweise 30°C bis 40°C, insbesondere 38°. Der Sollwertbereich beträgt beispielsweise eine Temperatur von mehr als 30°C oder 15°C bis 40°C, insbesondere 20°C bis 25°C.

Optional ist die Steuerungs-/ Regelungseinrichtung 34 derart ausgebildet, dass sie bei einer Abweichung der Temperatur von dem Sollwert oder dem Sollbereich, die Frequenz und /oder die Amplitude der Vibratoren 20, 22 erhöht oder verringert. Vorzugsweise wird bei einem Unterschreiten des Sollwertes oder des Sollbereichs die Frequenz und /oder die Amplitude der Vibratoren 20, 22 erhöht und bei einem Überschreiten des Sollwertes oder des Sollbereichs die Frequenz und /oder die Amplitude der Vibratoren 20, 22 verringert.

Die Steuerungs-/ Regelungseinrichtung 34 ist vorzugsweise derart ausgebildet, dass sie die Vibratoren 20, 22 gemäß eines vorabbestimmten und in der Steuerungs-/ Regelungseinrichtung 34 hinterlegten Mischprogramms steuert/ regelt.

Die Mischvorrichtung 10 weist optional eine nicht dargestellte Klopfeinrichtung auf, die vorzugsweise an der Aufnahmeeinrichtung 12 angebracht ist. Bei der Klopfeinrichtung handelt es sich beispielsweise um ein bewegbares Schlagstück. Beispielsweise ist das Schlagstück hydraulisch oder pneumatisch bewegbar. Die Klopfeinrichtung ist derart an der Aufnahmeeinrichtung 12 angebracht, dass sie diese mit Schlägen beaufschlagt, sodass vorzugsweise innerhalb des in der Aufnahmeeinrichtung 12 aufgenommenen Behälters ein Anbacken des Probematerials and er Behälterwand verhindert wird. Die Klopfeinrichtung ist vorzugsweise an der Aufnahmeeinrichtung 12 lösbar befestigt, sodass sie vorzugsweise lediglich im Bedarfsfall, in Abhängigkeit des zu mischenden Probematerials, an der Aufnahmeeinrichtung 12 befestigbar ist. Es ist ebenfalls denkbar, dass zusätzlich zu oder anstelle der Klopfeinrichtung zumindest einer oder beide Vibratoren 20, 22 als Klopfeinrichtungen ausgebildet sind. Des Weiteren kann der Pneumatikzylinder 14 zusätzlich als Klopfeinrichtung ausgebildet sein.

Des Weiteren ist die Steuerungs-/ Regelungseinrichtung 34 beispielsweise mit der Klopfeinrichtung verbunden und derart ausgebildet, dass sie die Intensität der Schläge der Klopfeinrichtung auf die Aufnahmeeinrichtung 12 vorzugsweise in Abhängigkeit des zu mischenden Probematerials steuert/ regelt.

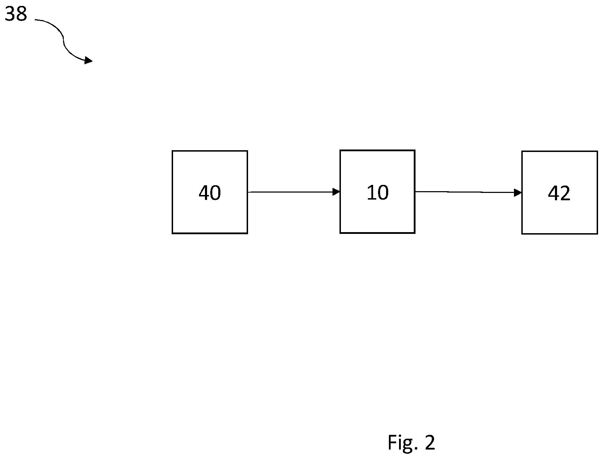

Fig. 2 zeigt eine Anlage 38 zur Bestimmung der Reaktivität eines hydraulischen Bindemittels. Die Anlage 38 umfasst eine Mischvorrichtung 10 wie vorangehend beschrieben, eine Dosiereinrichtung 40 zum Dosieren eines Probematerials in einen Behälter und ein Kalorimeter 42 zur Bestimmung der Reaktivität des Probematerials.

Die Mischvorrichtung kann in einen Automaten als Modul/Baugruppe integriert und automatisch beschickt werden. Hierbei wird die Apparatur an ein Rahmengestell des Automaten befestigt. In diesem Fall ist kein zusätzliches Gehäuse erforderlich. Alternativ kann die Mischvorrichtung sich auch in einem Tischgehäuse befinden und manuell beschickt werden. In einer weiteren Ausgestaltung kann die Mischapparatur auch in ein Kalorimeter integriert werden. Die Baugröße der Mischvorrichtung ist an den erforderlichen Probenbehälter, insbesondere die Probemenge anpassbar.

### Bezugszeichenliste

- 10: Mischvorrichtung
- 12: Aufnahmeeinrichtung
- 14: Pneumatikzylinder
- 16: zylinderförmige Aussparung
- 18: Rahmen
- 20: Vibrator
- 22: Vibrator
- 24a,b: Schwingungsdämpfer
- 26a,b: Schwingungsdämpfer
- 28a,b: Schwingungsdämpfer
- 30a,b: Schwingungsdämpfer
- 32: Temperiereinrichtung
- 34: Steuerungs-/Regelungseinrichtung
- 36: Temperaturmesseinrichtung
- 38: Anlage zur Bestimmung der Reaktivität eines hydraulischen Bindemittels
- 40: Dosiereinrichtung
- 42: Kalorimeter

## Patentansprüche

1. Mischvorrichtung (10) zur Vorbereitung eines Probematerials auf eine kalorimetrische Messung, aufweisend
einen Rahmen (18) und
eine mit dem Rahmen (18) verbundene Aufnahmeeinrichtung (12) zur Aufnahme eines mit dem Probematerial gefüllten Behälters, wobei zwischen der Aufnahmeeinrichtung (12) und dem Rahmen (18) ein Schwingungsdämpfer (24, 26, 28, 30) angebracht ist,
**dadurch gekennzeichnet, dass**
die Aufnahmeeinrichtung (12) mit zumindest zwei Vibratoren (20, 22) zur Vibration der Aufnahmeeinrichtung (12) verbunden ist, wobei die Vibratoren (20, 22) pneumatisch betrieben sind, und wobei einer der Vibratoren (20, 22) zur Erzeugung einer horizontalen und der andere der Vibratoren (20, 22) zur Erzeugung einer vertikalen Schwingung ausgebildet ist.

2. Mischvorrichtung (10) nach Anspruch 1, wobei die Aufnahmeeinrichtung (12) eine Temperiereinrichtung (32) zum Kühlen und/ oder Erwärmen des Behälters aufweist.

3. Mischvorrichtung (10) nach einem der vorangehenden Ansprüchen, wobei die Temperiereinrichtung (32) Leitungen zum Leiten eines Fluids aufweist.

4. Mischvorrichtung (10) nach einem der vorangehenden Ansprüchen, wobei die Mischvorrichtung (10) eine Temperaturmesseinrichtung (36) zur Ermittlung der Temperatur innerhalb des Behälters und/ oder der Aufnahmeeinrichtung (12) aufweist.

5. Mischvorrichtung (10) nach einem der vorangehenden Ansprüchen, wobei die Mischvorrichtung (10) eine Steuerungs-/ Regelungseinrichtung (34) aufweist, die mit dem Vibrator (20, 22) und/ oder der Temperiereinrichtung (32) verbunden ist und derart ausgebildet ist, die Temperatur der Temperiereinrichtung (32) und/ oder die Schwingungsamplitude und/ oder die Schwingungsfrequenz des Vibrators (20, 22) zu steuern/ regeln.

6. Mischvorrichtung (10) nach einem der vorangehenden Ansprüche, wobei die Aufnahmeeinrichtung (12) eine Klemmvorrichtung zum Fixieren des Behälters umfasst.

7. Mischvorrichtung (10) nach Anspruch 6, wobei die Klemmvorrichtung Klemmbacken und einen Pneumatikzylinder (14) aufweist, der zur Bewegung der Klemmbacken mit diesen verbunden ist.

8. Mischvorrichtung (10) nach Anspruch 7, wobei die Steuerungs-/ Regelungseinrichtung mit dem Pneumatikzylinder (14) zu dessen Steuerung/ Regelung verbunden ist.

9. Mischvorrichtung (10) nach Anspruch 5, wobei die Steuerungs-/Regelungseinrichtung (34) mit der Temperaturmesseinrichtung (36) verbunden und derart ausgebildet ist, dass sie die Temperatur der Temperiereinrichtung (32) und/ oder die Schwingungsamplitude und/ oder die Schwingungsfrequenz des Vibrators (20, 22) in Abhängigkeit der ermittelten Temperatur steuert/ regelt.

10. Mischvorrichtung (10) nach einem der vorangehenden Ansprüche, wobei die Mischvorrichtung (10) einen pneumatischen Klopfer aufweist, der an dem Behälter oder in der Aufnahmeeinrichtung (12) angeordnet ist.

11. Anlage (38) zur Bestimmung der Reaktivität eines Probematerials aufweisend
eine Dosiereinrichtung (40) zum Dosieren des Probematerials in einen Behälter, eine Mischvorrichtung (10) nach einem der vorangehenden Ansprüche, zur Aufnahme des Behälters und zum Mischen des Probematerials in dem Behälter und
ein Kalorimeter (42) zur Ermittlung der Reaktivität des gemischten Probematerials.

12. Verfahren zum Ermitteln der Reaktivität eines Probematerials aufweisend die Schritte:
Dosieren des Probematerials in einen Behälter,
Fixieren des Behälters in der Aufnahmeeinrichtung (12) einer Mischvorrichtung (10) nach einem der Ansprüche 1 bis 10,
Vibrieren der Aufnahmeeinrichtung (12) mittels der Vibratoren (20, 22) der Mischvorrichtung (10),
Zuführen des Behälters zu einem Kalorimeter (42) und
Ermitteln der Reaktivität des Probematerials in dem Behälter.

13. Verfahren nach Anspruch 12, wobei nach dem Dosieren des Probematerials in den Behälter zusätzlich eine Anregerflüssigkeit in den Behälter dosiert wird.

## Claims

1. Mixing device (10) for preparing a sample material for a calorimetric measurement, comprising
a frame (18) and
a receiving device (12) connected to the frame (18) for receiving a container filled with the sample material,
wherein a vibration damper (24, 26, 28, 30) is mounted between the mounting device (12) and the frame (18)**characterized in that**
the receiving device (12) is connected to at least two vibrators (20, 22) for vibrating the receiving device (12), wherein the vibrators (20, 22) are pneumatically operated, and wherein one of the vibrators (20, 22) is designed to generate a horizontal vibration and the other of the vibrators (20, 22) is designed to generate a vertical vibration.

2. Mixing device (10) according to claim 1, wherein the receiving device (12) has a temperature control device (32) for cooling and/or heating the container.

3. A mixing device (10) according to any one of the preceding claims, wherein the temperature control device (32) comprises conduits for conducting a fluid.

4. Mixing device (10) according to one of the preceding claims, wherein the mixing device (10) comprises a temperature measuring device (36) for determining the temperature inside the container and/or the receiving device (12).

5. Mixing device (10) according to one of the preceding claims, wherein the mixing device (10) has a control device (34) which is connected to the vibrator (20, 22) and/or the temperature control device (32) and is designed to open loop/ closed loop control the temperature of the temperature control device (32) and/or or the oscillation amplitude and/or the oscillation frequency of the vibrator (20, 22).

6. Mixing device (10) according to one of the preceding claims, wherein the receiving device (12) comprises a clamping device for fixing the container.

7. The mixing device (10) according to claim 6, wherein the clamping device comprises clamping jaws and a pneumatic cylinder (14) connected to the clamping jaws for moving the clamping jaws.

8. Mixing device (10) according to claim 7, wherein the control device is connected to the pneumatic cylinder (14) for its open loop/ closed loop control.

9. Mixing device (10) according to claim 5, wherein the control device (34) is connected to the temperature measuring device (36) and is designed such that it open loop/ closed loop controls the temperature of the temperature control device (32) and/or the oscillation amplitude and/or the oscillation frequency of the vibrator (20, 22) as a function of the determined temperature.

10. Mixing device (10) according to one of the preceding claims, wherein the mixing device (10) comprises a pneumatic knocker arranged on the container or in the receiving device (12).

11. Apparatus (38) for determining the reactivity of a sample material comprising
a dosing device (40) for dosing the sample material into a container,
a mixing device (10) according to one of the preceding claims, for receiving the container and for mixing the sample material in the container, and
a calorimeter (42) for determining the reactivity of the mixed sample material.

12. A method for determining the reactivity of a sample material comprising the steps of:
Dosing the sample material into a container,
Fixing the container in the receiving device (12) of a mixing device (10) according to any one of claims 1 to 10,
Vibrating the receiving device (12) by means of the vibrators (20, 22) of the mixing device (10),
Feeding the container to a calorimeter (42) and
Determine the reactivity of the sample material in the container.

13. The method according to claim 12, wherein after dosing the sample material into the container, an exciter liquid is additionally dosed into the container.

## Revendications

1. Dispositif de mélange (10) pour la préparation d'un échantillon de matériau pour une mesure calorimétrique, comprenant
un cadre (18) et
un dispositif de réception (12) relié au cadre (18) pour recevoir un récipient rempli de l'échantillon,
dans lequel un amortisseur de vibrations (24, 26, 28, 30) est monté entre le dispositif de montage (12) et le cadre (18), **caractérisé par le fait que** le dispositif de réception (12) est relié à au moins deux vibrateurs (20, 22) pour faire vibrer le dispositif de réception (12), les vibrateurs (20, 22) étant actionnés pneumatiquement, et l'un des vibrateurs (20, 22) étant conçu pour générer une vibration horizontale et l'autre des vibrateurs (20, 22) étant conçu pour générer une vibration verticale.

2. Dispositif de mélange (10) selon la revendication 1, dans lequel le dispositif de réception (12) comporte un dispositif de contrôle de la température (32) pour refroidir et/ou chauffer le récipient.

3. Dispositif de mélange (10) selon l'une quelconque des revendications précédentes, dans lequel le dispositif de contrôle de la température (32) comprend des conduits pour la conduite d'un fluide.

4. Dispositif de mélange (10) selon l'une des revendications précédentes, dans lequel le dispositif de mélange (10) comprend un dispositif de mesure de la température (36) pour déterminer la température à l'intérieur du récipient et/ou du dispositif de réception (12).

5. Dispositif de mélange (10) selon l'une des revendications précédentes, dans lequel le dispositif de mélange (10) comporte un dispositif de commande (34) qui est relié au vibrateur (20, 22) et/ou au dispositif de contrôle de la température (32) et qui est conçu pour contrôler en boucle ouverte/fermée la température du dispositif de contrôle de la température (32) et/ou l'amplitude de l'oscillation et/ou la fréquence de l'oscillation du vibrateur (20, 22).

6. Dispositif de mélange (10) selon l'une des revendications précédentes, dans lequel le dispositif de réception (12) comprend un dispositif de serrage pour fixer le récipient.

7. Dispositif de mélange (10) selon la revendication 6, dans lequel le dispositif de serrage comprend des mâchoires de serrage et un cylindre pneumatique (14) relié aux mâchoires de serrage pour déplacer les mâchoires de serrage.

8. Dispositif de mélange (10) selon la revendication 7, dans lequel le dispositif de contrôle est connecté au cylindre pneumatique (14) pour son contrôle en boucle ouverte/fermée.

9. Dispositif de mélange (10) selon la revendication 5, dans lequel le dispositif de commande (34) est relié au dispositif de mesure de la température (36) et est conçu de manière à contrôler en boucle ouverte/fermée la température du dispositif de contrôle de la température (32) et/ou l'amplitude d'oscillation et/ou la fréquence d'oscillation du vibrateur (20, 22) en fonction de la température déterminée.

10. Dispositif de mélange (10) selon l'une des revendications précédentes, dans lequel le dispositif de mélange (10) comprend un heurtoir pneumatique disposé sur le récipient ou dans le dispositif de réception (12).

11. Appareil (38) pour déterminer la réactivité d'un matériau d'échantillonnage comprenant
un dispositif de dosage (40) pour doser le matériau d'échantillonnage dans un récipient,
un dispositif de mélange (10) selon l'une des revendications précédentes, destiné à recevoir le récipient et à mélanger l'échantillon dans le récipient, et un calorimètre (42) pour déterminer la réactivité de l'échantillon mélangé.

12. Méthode de détermination de la réactivité d'un matériau d'échantillonnage comprenant les étapes suivantes
Doser le matériau d'échantillonnage dans un récipient,
Fixer le récipient dans le dispositif de réception (12) d'un dispositif de mélange (10) selon l'une quelconque des revendications 1 à 10,
Faire vibrer le dispositif de réception (12) au moyen des vibrateurs (20, 22) du dispositif de mélange (10),
Alimenter un calorimètre (42) avec le récipient et
Déterminer la réactivité de l'échantillon dans le récipient.

13. Méthode selon la revendication 12, dans laquelle, après avoir dosé le matériau d'échantillonnage dans le récipient, un liquide excitateur est également dosé dans le récipient.
